# EUROPEAN PATENT SPECIFICATION

(11) **EP 1 545 623 B1**
(45) Date of publication and mention of the grant of the patent: **09.05.2007**
(21) Application number: 03778269.5
(22) Date of filing: 25.09.2003
(51) Int. Cl.: A61K 47/48

(54) **CONJUGATES OF INSULIN-LIKE GROWTH FACTOR BINDING PROTEIN-4 AND POLY (ETHYLENE GLYCOL)**
POLY(ETHYLEN GLYKOL)-KONJUGATE VON INSULINÄHNLICHEM WACHSTUMFAKTOR BINDENDEN PROTEIN-4
CONJUGUES DE PROTEINE DE LIAISON DU FACTEUR DE CROISSANCE INSULINOMIMETIQUE 4 ET DE POLY (ETHYLENE GLYCOL)

(30) Priority: 27.09.2002 EP 02021844
(43) Date of publication of application: 29.06.2005
(73) Proprietor: F. HOFFMANN-LA ROCHE AG, 4070 Basel (CH)
(72) Inventor: LANG, Kurt, 82377 Penzberg (DE); SCHAUBMAR, Andreas, 81375 Muenchen (DE); SCHUMACHER, Ralf, 82377 Penzberg (DE)
(74) Representative: Schreiner, Siegfried
(86) International application number: PCT/EP2003/010658
(87) International publication number: WO 2004/028568

(56) References cited:
- VAN DEN BERG C L ET AL: "Polyethylene Glycol Conjugated Insulin-like Growth Factor Binding Protein-1 (IGFBP-1) Inhibits Growth of Breast Cancer in Athymic Mice" EUROPEAN JOURNAL OF CANCER, PERGAMON PRESS, OXFORD, GB, vol. 33, no. 7, June 1997 (1997-06), pages 1108-1113, XP004282806 ISSN: 0959-8049 cited in the application
- VERONESE F M: "Peptide and protein PEGylation - a review of problems and solutions" BIOMATERIALS, ELSEVIER SCIENCE PUBLISHERS BV., BARKING, GB, vol. 22, no. 5, 1 March 2001 (2001-03-01), pages 405-417, XP004227886 ISSN: 0142-9612 cited in the application
- FRANCIS G E ET AL: "PEGYLATION OF CYTOKINES AND OTHER THERAPEUTIC PROTEINS AND PEPTIDES: THE IMPORTANCE OF BIOLOGICAL OPTIMISATION OF COUPLING TECHNIQUES" INTERNATIONAL JOURNAL OF HEMATOLOGY, ELSEVIER SCIENCE PUBLISHERS, NL, vol. 68, no. 1, July 1998 (1998-07), pages 1-18, XP000791226 ISSN: 0925-5710

## Description

This invention relates to conjugates of insulin-like growth factor binding protein-4 (IGFBP-4) with poly(ethylene glycol) (PEG), pharmaceutical compositions containing such conjugates, and methods for the production and methods of use of such conjugates.

### Background of the Invention

The insulin-like growth factors (IGFs) are mitogens that play a pivotal role in regulating cell proliferation, differentiation and apoptosis. Six IGF-binding proteins (IGFBPs) can influence the actions of IGFs (Yu, H., and Rohan, T., J. Natl. Cancer Inst. 92 (2000) 1472-1489).

Mature human IGFBP-4 is described in the literature as a monomeric protein of 24 kDa and consists of 237 amino acids. The molecular weight of the protein calculated from the amino acid sequence is 26 kDa. Its biological role is reviewed in Yu, H., and Rohan, T., J. Natl. Cancer Inst. 92 (2000) 1472-1489. Conover, C.A., et al., in J. Biol. Chem. 270 (1995) 4395-4400, describe protease-resistant mutants of IGFBP-4. All four IGFBP-4 mutants around the putative cleavage site at Met135-Lys136 and the wildtype protein bind IGFs with equivalent affinities. Resistance of IGFBP-4 to proteolytic cleavage is also achieved by deletion of amino acids 121-141 as described by Miyakoshi, N., et al. in Endocrinology 142 (2001) 2641-2648. Byun, D., et al., in J. Endocrinology 169 (2001) 135-143, determined several regions involved in IGF binding by IGFBP-4. Deletion of segments Leu72-Ser 91 or Leu72-His74 results in loss of IGF binding. Also mutation of certain cysteine residues in the N- and the C-terminal domain significantly reduces the binding of IGFs.

IGFBP-4 was first isolated from medium conditioned by human osteosarcome TE-89 cells (Mohan, S., et al., Proc. Natl. Acad. Sci. USA 86 (1989) 8338-8342). IGFBP-4 is known to exist naturally as a non-glycosylated form with an apparent molecular weight of 24 kDa or in the glycosylated form weighing 28 kDa. Recombinant IGFBP-4 was produced by expression in several eukaryotic and prokaryotic systems. Human IGFBP-4 was produced by expression in E. coli as a fusion protein with glutathione S-transferase (Honda, Y., et al., J. Clin. Endocrinol. Metab. 81 (1996) 1389-1396) or as a fusion protein with a hexahistidine tag (Qin, X., et al., J. Biol. Chem. 273 (1998) 23509-23516) or by expression in yeast as ubiquitin fusion protein (Kiefer, M. C., et al., J. Biol. Chem. 267 (1992) 12692-12699). The sequence of human IGFBP-4 is described in detail in the SwissProt Database (http://www.expasy.ch) and identified by the Accession No. P 22692. The amino acid positions described in the following refer to the sequence of the mature forms of IGFBP-4 (sequence after removal of the signaling peptide starts with amino acid in position 1) or refer to the numbering used in the cited references.

IGFBP-4 inhibits the in vitro IGF-stimulated bone cell proliferation (Mohan, S., et al., Proc. Natl. Acad. Sci. USA 86 (1989) 8338-8342), the IGF-mediated growth of chick cartilage (Schiltz, P. M., et al., J. Bone Mineral Res. 8 (1993) 391-396) and the growth of HT-29 cells (Culouscou, J. M., and Shoyab, M., Cancer Res. 51 (1991) 2813-2819). Overexpression of IGFBP-4 in the malignant M 12 prostate epithelial cell line reduces the IGF-induced proliferation of the cells, inhibits colony formation in soft agar, increases apoptosis in response to 6-hydroxyurea and delays the formation of tumors by the transformed cells (Damon, S. E., et al., Endocrinology 139 (1998) 3456-3464). Overexpression of IGFBP-4 in human colorectal carcinoma cells reduces the proliferation of these cells and suppresses colony formation (Diehl, D., et al., J. Cancer Res. Clinical Oncol. 127, Suppl.1 (2001) S54).

Covalent modification of proteins with poly(ethylene glycol) (PEG) has proven to be a useful method to extend the circulating half-lives of proteins in the body (Hershfield, M.S., et al., New England Journal of Medicine 316 (1987) 589-596; and Meyers, F.J., et al., Clin. Pharmacol. Ther. 49 (1991) 307-313). Other advantages of PEGylation are an increase of solubility and a decrease in protein immunogenicity (Katre, N.V., J. Immunol. 144 (1990) 209-213). A common method for the PEGylation of proteins is the use of poly(ethylene glycol) activated with amino-reactive reagents like N-hydroxysuccinimide (NHS). With such reagents poly(ethylene glycol) is attached to the proteins at free primary amino groups such as the N-terminal α-amino group and the ε-amino groups of lysine residues. However, a major limitation of this approach is that proteins typically contain a considerable amount of lysine residues and therefore the poly(ethylene glycol) groups are attached to the protein in a non-specific manner at all of the free ε-amino groups, resulting in a heterologous product mixture of random PEGylated proteins. Therefore, many NHS-PEGylated proteins are unsuitable for commercial use because of low specific activity. Inactivation results from covalent modification of one or more lysine residues or the N-terminal amino residue required for biological activity or from covalent attachment of the poly(ethylene glycol) residues near or at the active site of the protein. For example, it was found that modification of human growth hormone using NHS-PEGylation reagents reduces the biological activity of the protein by more than 10-fold (Clark, R., et al., J. Biol. Chem. 271 (1996) 21969-21977). Human growth hormone contains 9 lysines in addition to the N-terminal amino acid. Certain of these lysines are located in regions of the protein known to be critical for receptor binding (Cunningham, B.C., et al., Science 254 (1991) 821-825). In addition, the modification of erythropoietin by the use of amino-reactive poly(ethylene glycol) reagents results also in a nearly complete loss of biological activity (Wojchowski, D.M., et al., Biochim. Biophys. Acta 910 (1987) 224-232). Covalent modification of Interferon-α2 with amino-reactive PEGylation reagents results in 40-75% loss of bioactivity (US Patent No. 5,382,657). A similar modification of G-CSF results in greater than 60% loss of activity (Tanaka, H., et al., Cancer Res. 51 (1991) 3710-3714) and of Interleukin-2 in greater than 90% loss of bioactivity (Goodson, R. J., and Katre, N. V., BioTechnology 8 (1990) 343-346).

Van den Berg, C.L., et al. (Europ. J. Cancer 33 (1997) 1108-1113; and WO 94/22466) covalently coupled cysteine-reactive poly(ethylene glycol) (20 kDa) to IGFBP-1, which leads to a prolonged serum half-life of 13.6 h. As described in WO 94/22466 it is believed that amino acids in the middle domain of IGFBP-1 can be substituted by cysteine for specific PEGylation without interference with the IGF binding and inhibition. Amino acids in positions 98 and 101 were exchanged against cysteine because Serine 101 is a natural major phosphoylation site, exposed on the protein surface and not involved in binding to IGFs. The 20 kDa monoPEGylated IGFBP-1 shows a comparable but no improved in vitro activity to wild-type IGFBP-1 regarding inhibition of tumor cell growth. According to van den Berg, C.L., et al., Europ. J. Cancer 33 (1997) 1108-1113, their PEGylated IGFBP-1 in vivo may not be able to inhibit IGF action directly on the tumor cell.

It is an object of the present invention to provide an improved IGFBP derivative with inhibitory efficacy on tumor growth and prolonged half life in vivo, which can preferably be administered as only a few bolus applications per week and which is capable of suppressing tumor growth, angiogenesis and/or metastasis.

### Summary of the Invention

It has surprisingly been found that 30 kDa to 40 kDa PEGylated (30-40 kDa PEGylated IGFBP-4), preferably monoPEGylated, IGFBP-4 according to the invention has superior properties in regard to therapeutic applicability in tumor treatment such as suppressing tumor growth, angiogenesis and/or metastasis in vivo, which cannot be found for IGFBP-4 alone or for lower weight PEGylated IGFBP-4. In addition, the conjugates according to the invention avoid undesired side effects in vivo such as alteration of normal kidney cells found for lower weight PEGylated IGFBP-4.

The present invention provides a conjugate consisting of an insulin-like growth factor binding protein-4 (IGFBP-4) and one or two poly(ethylene glycol) group(s), said poly(ethylene glycol) group(s) having an overall molecular weight of from about 30 to 40 kDa. Preferably, the poly(ethylene glycol) group(s) are conjugated to IGFBP-4 via primary amino group(s) (amino-reactive PEGylation). It is also preferred that the conjugate is a monoPEG-IGFBP-4 conjugate. It is particularly preferred that the conjugate is a mono-N-terminal PEG-IGFBP-4 conjugate coupled via the N-terminal amino group of IGFBP-4.

Also preferred are conjugates that include a branched PEG.

The invention further comprises methods for the production of the conjugates according to the invention.

The invention further comprises pharmaceutical compositions containing a conjugate according to the invention.

The invention further comprises methods for the production of pharmaceutical compositions containing a conjugate according to the invention.

The invention further comprises the use of a conjugate according to the invention for the preparation of a medicament for the treatment of cancer, preferably pancreatic cancer.

The invention further comprises methods for the treatment of human cancer (e.g. breast, lung, prostate or colon cancer), preferably pancreatic cancer, characterized in that a pharmaceutically effective amount of 30-40 kDa PEGylated IGFBP-4 is administered to a patient in need of such treatment, preferably in one to seven bolus applications per week.

### Detailed Description of the Invention

"Amino-reactive PEGylated IGFBP-4" or "amino-reactive PEGylation" as used herein means that IGFBP-4 is covalently bonded to one, two or three poly(ethylene glycol) groups by amino-reactive coupling to the IGFBP-4 molecule. The PEG groups can be attached at different sites of the IGFBP-4 molecule that are primary amino groups, preferably at the most reactive sites, e.g., the ε-amino groups of the lysine side chains or the N-terminal α-amino group. Due to the synthesis method used, PEGylated IGFBP-4 can consist of a mixture of mono-and/or diPEGylated IGFBP-4, whereby the sites of PEGylation can be different in different molecules or can be substantially homogeneous in regard to the amount of poly(ethylene glycol) side chains per molecule and/or the site of PEGylation in the molecule.

Amino-reactive PEGylation as used herein designates a method of randomly attaching poly(ethylene glycol) chains to primary amino group(s) of the target protein IGFBP-4 by the use of reactive (activated) poly(ethylene glycol), preferably by the use of N-hydroxysuccinimidyl esters of, preferably, methoxypoly(ethylene glycol). The coupling reaction preferentially attaches poly(ethylene glycol) to reactive primary amino groups like the ε-amino groups of lysine residues or the α-amino group of the N-terminal amino acid of IGFBP-4. Such amino group conjugation of PEG to proteins is well known in the art. For example, review of such methods is given by Veronese, F.M., Biomaterials 22 (2001) 405-417. According to Veronese, the conjugation of PEG to primary amino groups of proteins can be performed by using activated PEGs which perform an alkylation of said primary amino groups. For such a reaction, activated alkylating PEGs, for example PEG aldehyde, PEG-tresyl chloride or PEG epoxide can be used. Further useful reagents are acylating PEGs such as hydroxysuccinimidyl esters of carboxylated PEGs or PEGs in which the terminal hydroxy group is activated by chloroformates or carbonylimidazole. Further useful PEG reagents are PEGs with amino acid arms. Such reagents can contain the so-called branched PEGs, whereby at least two identical or different PEG molecules are linked together by a peptidic spacer (preferably lysine) and, for example, bound to IGFBP-4 as activated carboxylate of the lysine spacer. Mono-N-terminal coupling is also described by Kinstler, O., et al., Adv. Drug Deliv. Rev. 54 (2002) 477-485.

The occurrence of several potentially reactive primary amino groups in the target protein (for IGFBP-4 there are 12 lysines + 1 terminal amino acid) leads to a series of PEGylated IGFBP-4 isomers that differ in the point of attachment of the poly(ethylene glycol) chain and will hereinafter be referred to as "positional isomers". The attachment site in a single IGFBP-4 molecule is not clearly predicted and for that reason referred to as "random". Nine of these twelve lysines (Lys 67, Lys 124, Lys 134, Lys 136, Lys 192, Lys 204, Lys 210, Lys 215 and Lys 223) are located in regions that are reported to be required for complex formation with IGF (Qin, X., et al., J. Biol. Chem. 273 (1998) 23509-23516). Therefore, a strong reduction of the affinity to IGF would be expected for randomly PEGylated IGFBP-4. Surprisingly, this was not the case for the conjugates according to the invention.

It is also preferred to attach the PEG groups to IGFBP-4 via thiol-reactive PEGylation. Thiol-reactive PEGylation as used herein designates a method of attaching poly(ethylene glycol) to a target protein (IGFBP-4 mutant) by the use of activated, thiol-reactive poly(ethylene glycol), preferably by the use of N-maleimide esters of, preferably, methoxypoly(ethylene glycol). The coupling reaction preferentially attaches poly(ethylene glycol) to Cys110 and/or Cys117. Such sulfhydryl conjugation of PEG to proteins is widely known in the state of the art. A review of such methods is also given by, for example, Veronese, F.M., Biomaterials 22 (2001) 405-417. According to Veronese, the conjugation of PEG to thiol groups of proteins can be performed by using thiol-activated PEGs. For such a reaction, activated thiol-reactive PEGs, for example PEG-orthopyridyl-disulfide, PEG-maleimide, PEG-vinylsulfone, and PEG-iodoacetamide, can be used.

The invention provides PEGylated forms of IGFBP-4 with improved properties. Such PEGylated IGFBP-4 conjugates contain one or two PEG groups linear or branched and randomly attached thereto, whereby the overall molecular weight of all PEG groups in the conjugate is about 30 to 40 kDa. It is obvious to a person skilled in the art that small deviations from this range of molecular weight are possible as long as the PEGylated IGFBP-4 does not show such a negative influence on normal kidney cells as described in Example 15 for PEG20-IGFBP-4. Also PEGylation of IGFBP-4 with PEG having molecular weights of more than 40 kDa results in antitumorigenic activity. However, it is expected that such activity decreases as the molecular weight increases due to reduced tumor penetration.

As used herein, "molecular weight" means the mean molecular weight of the PEG. The term "about" before a designated molecular weight indicates that in said PEG preparations, some molecules will weigh more and some less than the stated molecular weight.

The following PEGylated forms of IGFBP-4 are examples of and are contemplated embodiments of the conjugates of the invention:
- monoPEGylated IGFBP-4, wherein the PEG group has a molecular weight of 30 or 40 kDa;
- diPEGylated IGFBP-4, wherein the PEG groups have a molecular weight of about 20 kDa each;
and mixtures thereof.

"PEG or PEG group" according to the invention means a residue containing poly(ethylene glycol) as an essential part. Such a PEG can contain further chemical groups which are necessary for binding reactions; which results from the chemical synthesis of the molecule; or which is a spacer for optimal distance of the parts of the molecule from one another. In addition, such a PEG can consist of one or more

PEG side-chains which are linked together. PEG groups with more than one PEG chain are called multiarmed or branched PEGs. Branched PEGs can be prepared, for example, by the addition of polyethylene oxide to various polyols, including glycerol, pentaerythriol, and sorbitol. For example, a four-armed branched PEG can be prepared from pentaerythriol and ethylene oxide. Branched PEGs usually have 2 to 8 arms and are described in, for example, EP-A 0 473 084 and US Patent No. 5,932,462. Especially preferred are PEGs with two PEG side-chains (PEG2) linked via the primary amino groups of a lysine (Monfardini, C., et al., Bioconjug. Chem. 6 (1995) 62-69).

"Substantially homogeneous" as used herein means that the only PEGylated IGFBP-4 molecules produced, contained or used are those having one or two PEG group(s) attached. The preparation may contain small amounts of unreacted (i.e., lacking PEG group) protein. As ascertained by peptide mapping and N-terminal sequencing, one example below provides for the preparation which is at least 90% PEG-IGFBP-4 conjugate (preferably monoPEGylated) and at most 5 % unreacted protein. Isolation and purification of such homogeneous preparations of PEGylated IGFBP-4 can be performed by usual purification methods, preferably size exclusion chromatography.

"MonoPEGylated" as used herein means that IGFBP-4 is PEGylated at only one amino group per IGFBP-4 molecule, whereby only one PEG group is attached covalently at this site and the sites of attachment can vary within the monoPEGylated species. The monoPEGylated IGFBP-4 is at least 90% of the preparation, and most preferably, the monoPEGylated IGFBP-4 is 92%, or more, of the preparation, the remainder being unreacted (non-PEGylated) IGFBP-4. The monoPEGylated IGFBP-4 preparations according to the invention are therefore homogeneous enough to display the advantages of a homogeneous preparation, e.g., in a pharmaceutical application. The same applies to the diPEGylated species.

"Activated PEGs or activated PEG reagents" are well-known in the state of the art. Preferably there are used electrophilically activated PEGs such as alkoxybutyric acid succinimidyl esters of poly(ethylene glycol) ("lower alkoxy-PEG-SBA") or alkoxypropionic acid succinimidyl esters of poly(ethylene glycol) ("lower alkoxy-PEG-SPA") or N-hydroxysuccinimide activated PEGs. Any conventional method of reacting an activated ester with an amine to form an amide can be utilized. In the reaction of the activated PEG with IGFBP-4, the exemplified succinimidyl ester is a leaving group causing the amide formation. The use of succinimidyl esters to produce conjugates with proteins is disclosed in US Patent No. 5,672,662.

When the PEGylation reagent is combined with IGFBP-4, it is found that at a pH of about 7.0, a protein:PEG ratio of about 1:3, and a reaction temperature of from 20-25 °C, a mixture of mono-, di-, and trace amounts of the tri-PEGylated species are produced. When the protein:PEG ratio is about 1:1, primarily the monoPEGylated species is produced. By manipulating the reaction conditions (e.g., ratio of reagents, pH, temperature, protein concentration, time of reaction etc.), the relative amounts of the different PEGylated species can be varied.

MonoPEGylated IGFBP-4 can also be produced according to the methods described in WO 94/01451. WO 94/01451 describes a method for preparing a recombinant polypeptide with a modified terminal amino acid alpha-carbon reactive group. The steps of the method involve forming the recombinant polypeptide and protecting it with one or more biologically added protecting groups at the N-terminal alpha-amine and C-terminal alpha- carboxyl. The polypeptide can then be reacted with chemical protecting agents to selectively protect reactive side chain groups and thereby prevent side chain groups from being modified. The polypeptide is then cleaved with a cleavage reagent specific for the biological protecting group to form an unprotected terminal amino acid alpha-carbon reactive group. The unprotected terminal amino acid alpha-carbon reactive group is modified with the activated PEG reagents. The side chain protected terminally modified single copy polypeptide is then deprotected at the side chain groups to form a terminally modified recombinant single copy polypeptide. The number and sequence of steps in the method can be varied to achieve selective modification.

IGFBP-4 conjugates according to the invention may be prepared by covalently reacting a primary amino group of an IGFBP-4 protein with a bifunctional reagent to form an intermediate with an amide linkage and covalently reacting the intermediate containing amide linkage with an activated poly(ethylene glycol) derivative to form an IGFBP-4 protein conjugate. In the foregoing process, the bifunctional reagent is preferably N-succinimidyl-S-acetylthiopropionate or N-succinimidyl-S-acetylthioacetate, and the activated poly(ethylene glycol) derivative is preferably selected from the group consisting of iodo-acetyl-methoxy-PEG, methoxy-PEG-vinylsulfone, and methoxy-PEG-maleimide.

The IGFBP-4 conjugates may be prepared by amino-reactive covalent linking of thiol groups to IGFBP-4 ("activation") and coupling the resulting activated IGFBP-4 with a poly(ethylene glycol) (PEG) derivative. The first step comprises covalent linking of thiol groups via NH₂-groups of IGFBP-4. This activation of IGFBP-4 is performed with bifunctional reagents which carry a protected thiol group and an additional reactive group, such as active esters (e.g., a succinimidylester), anhydrides, esters of sulphonic acids, halogenides of carboxylic acids and sulphonic acids, respectively. The thiol group is protected by groups known in the art, e.g., acetyl groups. These bifunctional reagents are able to react with the ε-amino groups of the lysine amino acids by forming an amide linkage. The preparation of the bifunctional reagents is known in the art. Precursors of bifunctional NHS esters are described in DE 39 24 705, while the derivatization to the acetylthio compound is described by March, J., Advanced Organic Chemistry (1977) 375-376. The bifunctional reagent SATA is commercially available (Molecular Probes, Eugene, OR, USA and Pierce, Rockford, IL) and described in Duncan, R.J., Anal. Biochem. 132 (1983) 68-73.

The number of thiol groups to be added to an IGFBP-4 molecule can be selected by adjusting the reaction parameters, i.e., the protein (IGFBP-4) concentration and the protein/bifunctional reagent ratio. Preferably, the IGFBP-4 is activated by covalently linking from 1 to 5 thiol groups per IGFBP-4 molecule, more preferably from 1.5 to 3 thiol groups per IGFBP-4 molecule. These ranges refer to the statistical distribution of the thiol group over the IGFBP-4 protein population.

The reaction is carried out, for example, in an aqueous buffer solution, pH 6.5-8.0, e.g., in 10 mM potassium phosphate, 300 mM NaCl, pH 7.3. The bifunctional reagent may be added in DMSO. After completion of the reaction, preferably after 30 minutes, the reaction is stopped by addition of lysine. Excess bifunctional reagent may be separated by methods known in the art, e.g., by dialysis or column filtration. The average number of thiol groups added to IGFBP-4 can be determined by photometric methods described in, for example, Grasetti, D.R,. and Murray, J.F. in J. Appl. Biochem. Biotechnol. 119 (1967) 41-49.

The above reaction is followed by covalent coupling of an activated poly(ethylene glycol) (PEG) derivative. Suitable PEG derivatives are activated PEG molecules with an average molecular weight of from about 15 to about 40 kDa, depending on whether mono- or diPEGylated product is desired.

Activated PEG derivatives are known in the art and are described in, for example, Morpurgo, M., et al. J. Bioconjug. Chem. 7 (1996) 363-368 for PEG-vinylsulfone. Linear chain and branched chain PEG species are suitable for the preparation of the compounds according to the invention. Examples of reactive PEG reagents are iodo-acetyl-methoxy-PEG and methoxy-PEG-vinylsulfone. The use of these iodo-activated substances is known in the art and is described e.g. by Hermanson, G.T., in Bioconjugate Techniques, Academic Press, San Diego (1996) p. 147-148.

A preferred method for cysteine specific PEGylation as used herein designates a method of attaching poly(ethylene glycol) chains to a target polypeptide (IGFBP-4) by the use of a 20 kDa methoxy-poly(ethylene glycol)-maleimide or branched 40 kDa PEG2-maleimide (=PEG-maleimide) (Shearwater Polymers, Inc.; Huntsville, Alabama) to a reduced sufhydryl group of the polypeptide chain of the protein. Native IGFBP-4 does not possess free cysteins because all cysteins are involved in the formation of disulfide bonds. Reduction of native IGFBP-4 in the presence of mild or low concentrations of reducing agents such as β-mercaptoethanol, dithiotreitol or TCEP results in selective opening of a disulfide bond and the exposure of reduced sulfhydryl groups which can be specifically modified with PEG-maleimide.

It is assumed that the disulfide bonds in the middle domain of IGFBP-4 are highly sensitive to reduction and therefore enables cysteine-specific PEGylation at cysteine 110 or cysteine 117. The specificity of the coupling reaction for cysteine 110 and 117 of IGFBP-4 was confirmed by peptide mapping of the isolated monoPEGylated IGFBP-4 and identification of the peptides by LC-MS mass spectrometry and sequencing of the peptide peaks. PEGylated forms of IGFBP-4 demonstrated a reduced peak area of the peptide containing the two cysteines.

Most preferably, the PEG species are activated by maleimide using (alkoxy-PEG-maleimide), such as methoxy-PEG-maleimide (MW 15,000 to 40,000; Shearwater Polymers, Inc.). The coupling reaction with alkoxy-PEG-maleimide takes place after in situ cleavage of the thiol protecting group in an aqueous buffer solution, e.g. 10 mM potassium phosphate, 300 mM NaCl, 2 mM EDTA, pH 6.2. The cleavage of the protecting group may be performed, for example, with hydroxylamine in DMSO at 25°C, pH 6.2 for about 90 minutes. For the PEG modification the molar ratio of activated IGFBP-4/alkoxy-PEG-maleimide should be from about 1:1 to about 1:6. The reaction may be stopped by addition of cysteine and reaction of the remaining thiol (-SH) groups with N-methylmaleimide or other appropriate compounds capable of forming disulfide bonds. Because of the reaction of any remaining active thiol groups with a protecting group such as N-methylmaleimide or other suitable protecting group, the IGFBP-4 proteins in the conjugates of this invention may contain such protecting groups. Generally the procedure described herein will produce a mixture of molecules having varying numbers of thiols protected by different numbers of the protecting group, depending on the number of activated thiol groups on the protein that were not conjugated to PEG-maleimide.

Whereas N-methylinaleimide forms the same type of covalent bond when used to block the remaining thiol-groups on the PEGylated protein, disulfide compounds will lead in an intermolecular sulfide/disulfide exchange reaction to a disulfide bridged coupling of the blocking reagent. Preferred blocking reagents for that type of blocking reaction are oxidized glutathione (GSSG), cysteine and cystamine. Whereas with cysteine no additional net charge is introduced into the PEGylated protein, the use of the blocking reagents GSSG or cystamine results in an additional negative or positive charge.

Thiol-reactive PEGylation of IGFBP-4 mutants can be performed according to the methods of the state of the art (see, e.g., WO 94/22466, and Veronese, F.M., Biomaterials 22 (2001) 405-417). Further activated PEG derivatives are known in the art and are described in, for example, Morpurgo, M., et al. J. Bioconjug. Chem. 7 (1996) 363-368 for PEG-vinylsulfone. Linear chain and branched chain PEG species are suitable for the preparation of the compounds according to the invention. Examples of reactive PEG reagents are iodo-acetyl-methoxy-PEG and methoxy-PEG-vinylsulfone. The use of these iodo-activated substances is known in the art and described e.g. by Hermanson, G.T., in Bioconjugate Techniques, Academic Press, San Diego (1996) p.147-148.

The further purification of the compounds according to the invention including the separation of mono- and/or diPEGylated IGFBP-4 species from higher PEGylated forms may be done by methods known in the art, e.g., column chromatography.

The percentage of mono-PEG conjugates as well as the ratio of mono- and di-PEG species can be controlled by pooling broader fractions around the elution peak to decrease the percentage of mono-PEG or narrower fractions to increase the percentage of mono-PEG in the composition. About ninety percent mono-PEG conjugates is a good balance of yield and activity. Sometimes compositions in which, for example, at least ninety-two percent or at least ninety-six percent of the conjugates are mono-PEG species (n equals 1) may be desired. In an embodiment of this invention the percentage of conjugates where n is 1 is from ninety percent to ninety-six percent.

### Pharmaceutical Formulations:

PEGylated IGFBP-4 can be administered as a mixture, or as the ion exchange chromatography or size exclusion chromatography separated different PEGylated species. The compounds of the present invention can be formulated according to methods for the preparation of pharmaceutical compositions which methods are known to the person skilled in the art. For the production of such compositions, PEGylated IGFBP-4 according to the invention is combined in a mixture with a pharmaceutically acceptable carrier, preferably by dialysis against an aqueous solution containing the desired ingredients of the pharmaceutical compositions. Such acceptable carriers are described, for example, in Remington's Pharmaceutical Sciences, 18^{th} edition, 1990, Mack Publishing Company, edited by Oslo et al. (e.g. pp. 1435-1712). Typical compositions contain an effective amount of the substance according to the invention, for example from about 0.1 to 100 mg/ml, together with a suitable amount of a carrier. The compositions may be administered parenterally.

The pharmaceutical formulations according to the invention can be prepared according to known methods in the art. Usually, solutions of PEGylated IGFBP-4 are dialyzed against the buffer intended to be used in the pharmaceutical composition and the desired final protein concentration is adjusted by concentration or dilution.

Such pharmaceutical compositions may be used for administration for injection or infusion and contain an effective amount of the monoPEGylated IGFBP-4 together with pharmaceutically acceptable diluents, preservatives, solubilizers, emulsifiers, adjuvants and/or carriers. Such compositions include diluents of various buffer contents (e.g. arginine, acetate, phosphate), pH and ionic strength, additives such as detergents and solubilizing agents (e.g. Tween^{™} 80/polysorbate, pluronic^{™} F68), antioxidants (e.g. ascorbic acid, sodium metabisulfite), preservatives (Timersol^{™}, benzyl alcohol) and bulking substances (e.g. saccharose, mannitol), incorporation of the material into particulate preparations of polymeric compounds such as polylactic acid, polyglycolic acid, etc. or into liposomes. Such compositions may influence the physical state stability rate of release and clearance of the monoPEGylated IGFBP-4 according to the invention.

### Dosages and Drug Concentrations:

Typically, in a standard cancer treatment regimen, patients are treated with dosages in the range between 0.01 to 3 mg of PEGylated IGFBP-4 per kg per day over a certain period of time, lasting from one day to about 30 days or even longer. Drug is applied as a single daily subcutaneous or i.v. or i.p. (intraperitoneal) bolus injection or infusion of a pharmaceutical formulation containing 0.1 to 100 mg PEGylated IGFBP-4 per ml. This treatment can be combined with any standard (e.g. chemotherapeutic) treatment, by applying PEGylated IGFBP-4 before, during or after the standard treatment. This results in an improved outcome compared to standard treatment alone.

### Brief Description of the Figures

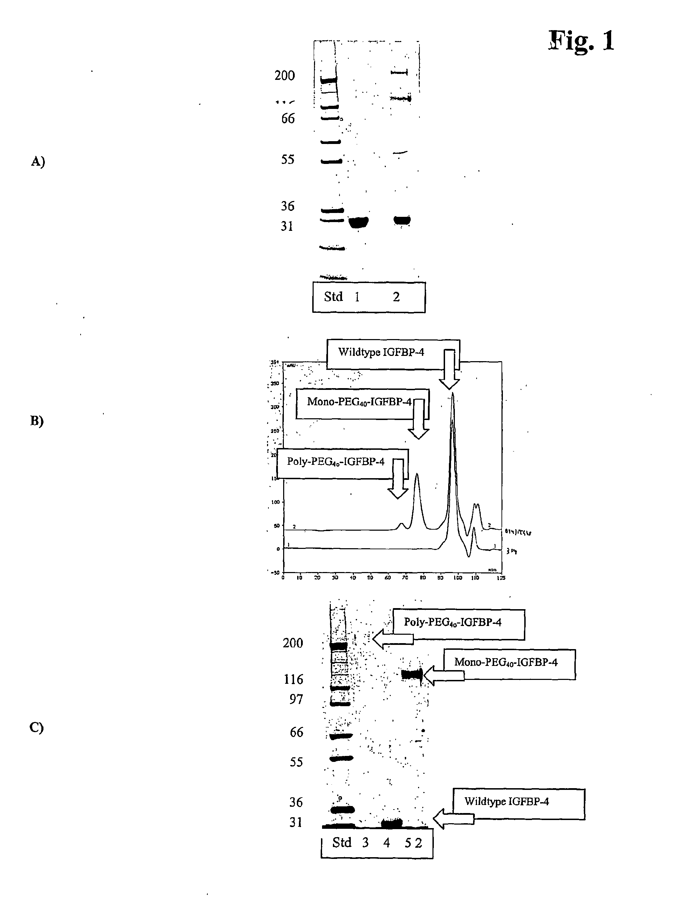

- Fig. 1:: PEGylation of IGFBP-4 with 40kDa PEG and separation of the PEGylated products by SEC.
A) Coomassie stained SDS-PAGE of starting material (lane 1) and outcome of the 40kDa PEGylation reaction. Std = Markl2 Molecular weight standard (Invitrogen); 1= IGFBP-4 wildtype (starting material); 2 = IGFBP-4 after PEGylation with 40kDa mPEG2.
B) Separation of the PEGylated products by size exclusion chromatography ("SEC"). SEC of random 40kDa PEGylated IGFBP-4 was performed on Superose 6 (Pharmacia) in 20mM Phosphate pH7.5, 500mM NaCl, flow rate 0.5ml/min.
C) Analysis of PEGylated products by SDS PAGE. Std = Markl2 Molecular weight standard (Invitrogen); 3 = polyPEG40-IGFBP-4; 4 = unPEGylated IGFBP-4; 5 = monoPEG40-IGFBP-4.

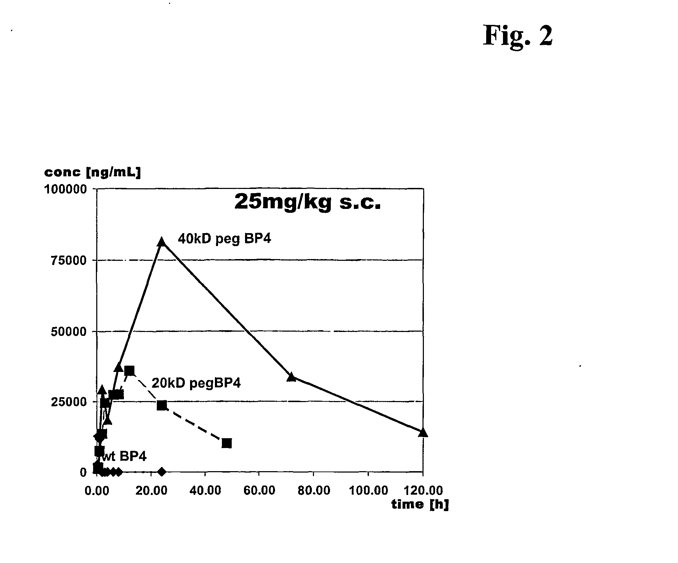

- Fig. 2:: Serum kinetics of mono40kDa-PEG-IGFBP-4 compared with mono20kDa-PEG-IGFBP-4 and unPEGylated IGFBP-4.
SCID mice were injected subcutaneously with a single dose of 1mg/200µl mono40kDa-PEG-IGFBP-4 or mono20kDa-PEG-IGFBP-4 or unPEGylated IGFBP-4 in PBS. Serum samples were collected in a time range from 0.5 to 120h after injection, as indicated, and analyzed for mono40kDa-PEG-IGFBP-4 or mono20kDa-PEG-IGFBP-4 or unPEGylated IGFBP-4 by Western Blotting with an anti-IGFBP-4 antibody (UBI) after affinity purification or by ELISA.

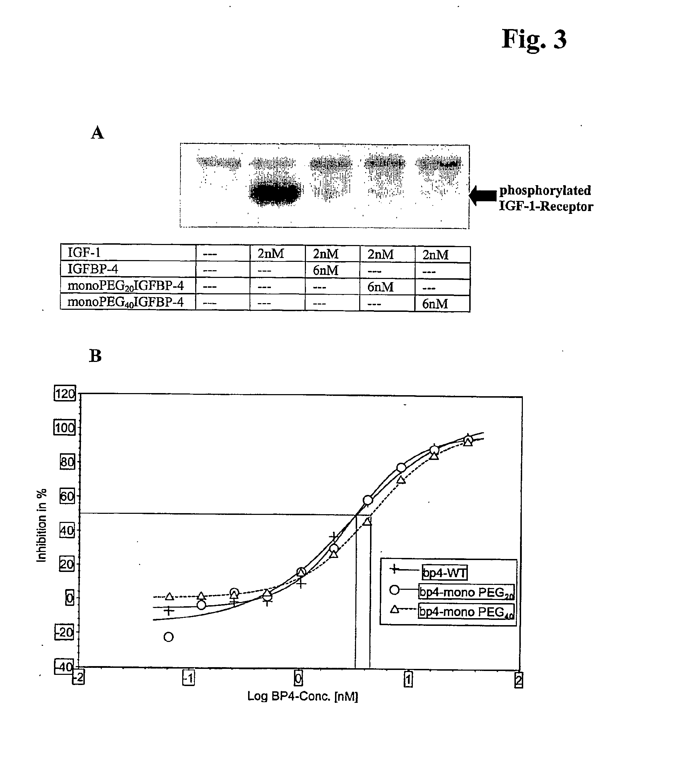

- Fig. 3:: Inhibition of IGF-I mediated phosphorylation of IGF-I receptor by IGFBP-4 derivatives.
A) Western blot analysis of IGF-IR phosphorylation by IGF-I in the absence and presence of IGFBP-4 derivatives.
   NIH3T3 cells overexpressing IGF-I-receptor were stimulated with 2nM IGF-I in the presence or absence of a threefold excess of IGFBP-4. After 10 minutes, cells were lysed and lysates subjected to a Western Blotting procedure with anti-phosphotyrosine antibodies to detect tyrosine phosphorylated IGF-I receptor. Mono20kDa-PEG-IGFBP-4 and mono40kDa-PEG-IGFBP-4 inhibited the receptor stimulation completely at concentrations of 6nM. In terms of inhibiting IGF-I induced IGF-I-receptor phosphorylation both PEGylated forms of IGFBP-4 proved to be as efficient as wildtype IGFBP-4.
B) Titration of IGF-I mediated phosphorylation of IGF-I receptor by IGFBP-4 derivatives.
   NIH3T3 cells overexpressing IGF-I-receptor were stimulated with 3.3nM IGF-I in the presence or absence of varying concentrations of IGFBP-4 derivatives. After 10 minutes, cells were lysed and lysates subjected to an ELISA based measurement of phosphorylated IGF-I-Receptor. MonoPEGylated IGFBP-4 (20 or 40kDa) and wildtype IGFBP-4 inhibited the receptor stimulation with an IC50 of 3nM.

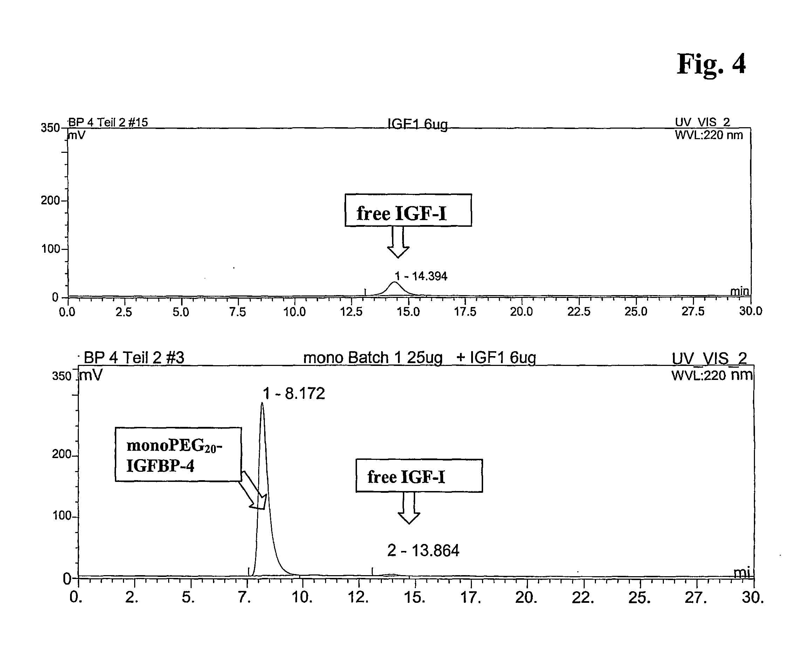

- Fig. 4:: IGF-binding of monoPEG20-IGFBP-4 determined by Size exclusion chromatography.
The binding abilities of IGFBP-4 or PEGylated isoforms thereof were determined by a size exclusion chromatography based assay. 70nmol (6µg) of IGF-I are injected on the column (HRP 75, Pharmacia; running conditions: 20mM sodium phosphate pH 7.4, 500mM NaCl, 1ml/min) either alone or together with 96nmol mono20kDa-PEG-IGFBP-4 (equivalent to 25µg wildtype IGFBP-4) after a preincubation step (30min at room temperature). Free IGF-I is quantified by integrating the IGF-I peak of the chromatogram (Chromeleon, Dionex). The peak area is negatively correlated with the binding capacity of IGFBP-4. In the demonstrated experiment, more than 90% of IGF-I is bound by mono20kDa-PEG-IGFBP-4. Similar results were obtained with mono40kDa-PEG-IGFBP-4.

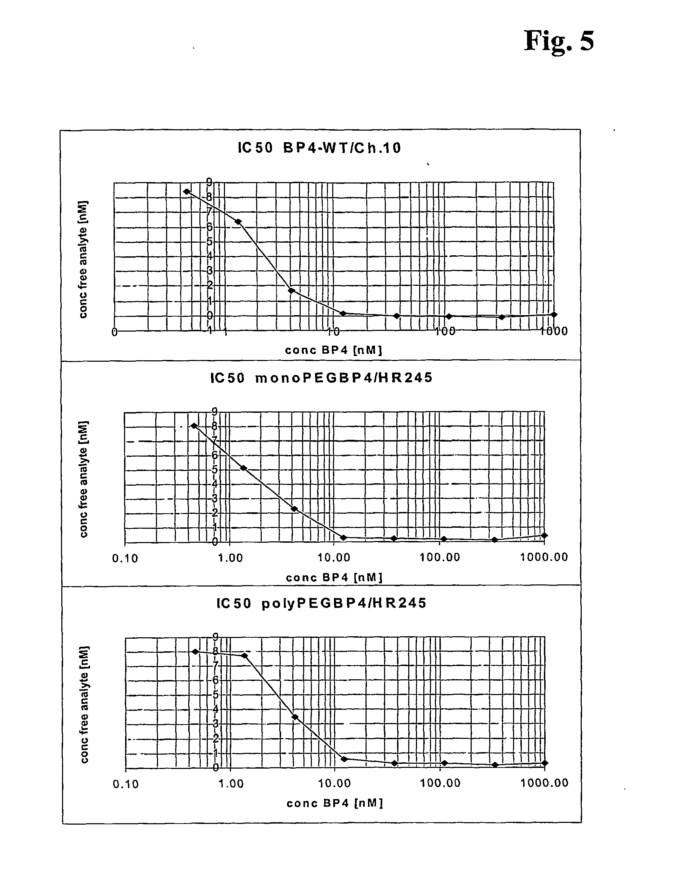

- Fig. 5:: Inhibition of the IGF-I binding to immobilized wildtype IGFBP-4 by wildtype, mono- and poly-20kDa random PEGylated IGFBP-4. Determination of IC50 values of wildtype, monoPEG-IGFBP-4 and polyPEG-IGFBP-4. For measuring IC50 values, wildtype IGFBP-4 was immobilized on a sensor chip surface. Binding of IGF-1 (10nM) to the immobilized IGFBP-4 was challenged by the addition of increasing concentrations of wildtype IGFBP-4, monoPEG20-IGFBP-4 and polyPEG20IGFBP-4 prior to chip contact. Competition of IGF-1 binding to the immobilized IGFBP-4 with mono- or polyPEGylated IGFBP-4 was found to be as efficient as with wildtype IGFBP. Similar results were obtained with 40kDa-PEG-IGFBP-4.

### Example 1

### Fermentation, renaturation and purification of IGFBP-4

Production of recombinant wildtype IGFBP-4 in E.coli or yeast was described for example by Miyakoshi, N., et al., Endocrinology 142(2001) 2641-2648, and by Kiefer, M.C., et al., J. Biol. Chem. 267 (1992) 12692-12699. Human recombinant IGFBP-4 is further commercially available (e.g. from GroPep Ltd.; Adelaide, Australia).

### Fermentation conditions:

Seed culture was performed in a 500ml Erlenmeyer-flask with a culture volume of 100 ml at 37°C on a shaking incubator for 7h. The main culture was carried out in a 10 1 fed-batch fermentation with an initial volume of 8 1. The pH of the culture medium (Springer-Yeast 50g/l, K₂HPO₄*₃H₂O₃ g/l, MgSO₄*₇H₂O 0.74g/1, glucose 4.0 g/l, ampicillin 100mg/l, kanamycinsulfate 50mg/l) was maintained at pH 6.8 + 0.3 by addition of an ammonia solution (12% w/v) as base and a glucosemonohydrate solution (75% w/v) as acid and carbon source. The dissolved oxygen level was sustained at or above 20% by supplying air at a rate of 1.0 vvm and altering the agitation speed (500 rpm-1000 rpm).

After the culture reached an optical density (OD) of 10 (measured at 580nm with UV-visible spectrometer) the feeding with a Springer-yeast solution (500 g/l) was started. The induction of the protein expression was carried out at an OD of 15 with 1mM Isopropyl-β-D-thiogalactopyranoside (IPTG). IGFBP-4 is predominantly expressed insoluble as inclusion bodies (approx. 80%).

Cultivation was continued up to 12h to an OD of 35 and then the cells were harvested by centrifugation (13.000 rpm for 30 min).

### Isolation and purification of inclusion bodies:

The cell pellet was suspended in 0.1 M Tris-HCl buffer (pH 7.0) containing 1mM MgSO4. After addition of 0.3 g lysozyme per 100g dry cell weight and 30 U benzonase per 1 g dry cell weight, the cell suspension was subjected to French press (1000 bar (14,500 psi), one cycle) for disruption. After disruption the suspension was diluted 1:2 with Brij-buffer (200ml/l Brij 30%, 1.5M NaCl, 0.1M EDTA, pH 7.0) and further stirred at room temperature for 30 minutes. To isolate the inclusion bodies the suspension was centrifuged at 13.000 rpm for 30min. The obtained pellet was resuspended with 0.1M Tris-HCl buffer (pH 6.5) containing 5mM EDTA and centrifuged again at 13.000 rpm for 30 min. The pellet containing the purified inclusion bodies was stored at -20°C until further purification of IGFBP-4.

### Solubilization, naturation and purification:

20g of inclusion bodies were solubilized in a buffer containing 8M guanidinium chloride, 100mM Tris, 5mM EDTA and 100mM DTE (pH 8.5). After solubilization, pH 2.5 was adjusted with HCl and the solubilisate was dialyzed against 6M guanidinium chloride, 5mM EDTA (pH 2.5). Protein content was analyzed by UV absorption. Naturation was performed at room temperature. The unfolded protein was diluted in two pulses (with a 5h interval) of 0.25mg protein per ml in a volume of 4500ml (0.6M arginine, 1mM EDTA, 3mM GSH, 1mM GSSG (pH 8.5)) to a final protein concentration of 0.5mg/ml. Naturation was completed over night.

The naturation sample was stocked up to 25% (NH4)2SO4 and centrifuged. The supernatant was dialyzed against 50mM sodium citrate, 100mM NaCl (pH 4.5) and brought to 0.8M (NH4)2SO4 and 0.2M arginine (by the addition of solid (NH4)2SO4 and dilution of a 1M arginine/HCl stock solution). After pH adjustment to pH 8.5 with NaOH, the sample was applied to a phenyl sepharose column (phenyl sepharose fast flow (Pharmacia); equilibrated with 20mM sodium phosphate, 100mM NaCl, 1M (NH4)2SO4 (pH 7.5)). The column was washed with equilibration buffer without (NH4)2SO4. Elution of IGFBP-4 was achieved in a gradient from 20mM sodium phosphate to 20mM sodium phosphate supplemented with 50% ethylene glycol and a post-elution wash with 20mM sodium phosphate, 100mM NaCl, 50% ethylene glycol, pH 7.5.

The eluate was pooled according to SDS-PAGE, diluted 1:2 with 50mM citrate pH 4.2 and applied on a S-sepharose column (Pharmacia). The column was washed with 20mM sodium phosphate pH 7.5 and elution was performed with a gradient to 20mM sodium phosphate, 600mM NaCl. IGFBP-4 was finally pooled on the basis of SDS-PAGE.

### Example 2

### 40kDa PEGylation of IGFBP-4 (random amino-reactive PEGylation)

40kDa PEGylation is achieved by reacting IGFBP-4 with mPEG2-NHS ester, a lysine derivative carrying two 20kDa PEG chains and a single reactive N-Hydroxysuccinimidyl ester (Shearwater Polymers, Inc.; Huntsville, Alabama, USA; thereafter named 40kDa mPEG2).

IGFBP-4 is PEGylated by the addition of an aqueous solution of 40kDa mPEG2 to a concentrated IGFBP-4 solution in PBS. 40kDa mPEG2 was added in a molar ratio of 2 molecules PEG per molecule IGFBP-4. The reaction was allowed to proceed at room temperature for 30 minutes and was finally quenched by adding 1M arginine solution (buffered to pH 8.0 with HCl) to a final concentration of 100mM.

The outcome of the PEGylation reaction was optimized for maximal production of monoPEGylated IGFBP-4 with simultaneous minimal consumption of mPEG2-NHS reagent by carefully titrating protein and PEG concentrations. For IGFBP-4, yields of the monoPEGylated isoforms is best at elevated protein concentrations (c=5mg/ml or higher) and a 2fold molar excess of PEGylation reagent.

### Example 3

### N-terminal PEGylation of IGFBP-4

N-terminal specific PEGylation as used herein designates a method of attaching poly(ethylene glycol) chains to a target polypeptide (IGFBP-4) by the use of a poly(ethylene glycol) aldehyde at acidic pH under reducing conditions. The coupling reaction preferentially attaches PEG-aldehyde to the N-terminal aminogroup of a polypeptide chain with little or no side reactions involving ε-amino groups of lysine.

Human IGFBP-4 was dialyzed against 20mM acetate buffer, pH4.5 and PEGylated by the addition of an aqueous solution of 40kDa or 20kDa PEG-aldehyde (Shearwater Polymers, Inc.; Huntsville, Alabama). PEG-aldehyde was added in a molar ration of 2 molecules PEG per molecule IGFBP-4. PEG-aldehyde forms a Schiff base with the N-terminal amino group which is subsequently (i.e. after one hour of incubation) reduced by the addition of sodium cyano borohydrid to a final concentration of 20mM. The reaction is allowed to proceed over night at room temperature.

The outcome of the PEGylation reaction was optimized for maximal production of N-terminally monoPEGylated IGFBP-4 with simultaneous minimal consumption of PEG-aldehyde reagent by carefully titrating protein and PEG concentrations. For IGFBP-4, yields of the N-terminally monoPEGylated isoforms are best at elevated peptide concentrations (c=1 mg/ml or higher) and a 1.5 molar excess of PEGylation reagent. Purification of monoPEGylated IGFBP-4 was performed as described for the random PEGylated protein. The specificity of the coupling reaction for the N-terminus of the protein was confirmed by N-terminal sequencing and peptide mapping of the isolated monoPEGylated IGFBP-4 by use of the endoproteinase Lys C (sequence grade; Roche Diagnostics GmbH; Germany) and identification of the peptides by LC-MS mass spectrometry.

### Example 4

### Cysteine specific PEGylation of wild-type IGFBP-4

Cysteine specific PEGylation as used herein designates a method of attaching poly(ethylene glycol) chains to a target polypeptide (IGFBP-4) by the use of a 20 kDa methoxy-poly(ethylene glycol)-maleimide or branched 40 kDa PEG2-maleimide (=PEG-maleimide) (Shearwater Polymers, Inc.; Huntsville, Alabama) to a reduced sufhydryl group of the polypeptide chain of the protein. Native IGFBP-4 does not possess free cysteins because all cysteins are involved in the formation of disulfide bonds. Reduction of native IGFBP-4 in the presence of mild or low concentrations of reducing agents such as β-mercaptoethanol, dithiotreitol or TCEP results in selective opening of a disulfide bond and the exposure of reduced sulfhydryl groups which can be specifically modified with PEG-maleimide.

IGFBP-4 was dialyzed at a concentration of 0.75 mg/ml against 20mM sodium phosphate, 150 mM NaCl, pH 7.2 and DTT or β-mercaptoethanol was added to concentrations of 30 to 1000 uM. The mixtures were incubated for 4 h and than an aqueous solution of 20 kDa PEG-maleimide was added to a concentration of 1.6 mg/ml. The reaction was stopped and analyzed after one hour by SDS-PAGE. MonoPEGylated IGFBP-4 was isolated from samples containing the highest proportion of this PEGylated derivative by methods described for random PEGylated IGFBP-4.

It is assumed that the disulfide bonds in the middle domain of IGFBP-4 are highly sensitive to reduction and therefore enables cysteine-specific PEGylation at cysteine110 or cysteine 117. The specificity of the coupling reaction for cysteine 110 and 117 of IGFBP-4 was confirmed by peptide mapping of the isolated monoPEGylated IGFBP-4 and identification of the peptides by LC-MS mass spectrometry and sequencing of the peptide peaks. PEGylated forms of IGFBP-4 demonstrated a reduced peak area of the peptide containing the two cysteines.

### Example 5

### Purification of 40kDa-PEG-IGFBP-4 isomers

Preparative separation of PEGylation products for biochemical and biological analysis is achieved by size exclusion chromatography on a sephacryl S 400 column (Pharmacia) in a running buffer consisting of 20mM sodium phosphate pH 7.5 supplemented with 500mM sodium chloride.

The 40kDa-PEG-IGFBP-4 isomers elute earlier in size exclusion chromatography as compared to the unmodified form. This is due to an increased hydrodynamic radius of the molecule.

Eluting fractions were further analyzed by SDS-PAGE. In SDS-PAGE proteins are separated according to their molecular weight. PEGylated forms of IGFBP-4 migrated more slowly than the wildtype protein. The speed of migration is inversely correlated with the amount of PEG attached to the protein. Separation was performed on NOVEX 4-12% NuPage gels in a MOPS SDS buffer system.

Products were combined to three pools which are designated as follows:
- Poly40kDa-PEG-IGFBP-4: a mixed population of PEGylation isoforms, consisting of more than 90% IGFBP-4 carrying two or more 40kDa mPEG2 residues. The theoretical molecular weight of the poly40kDa-PEG-IGFBP-4 is 86kDa and higher. Apparently they run at more than 200kDa in SDS-PAGE
- Mono40kDa-PEG-IGFBP-4: a more than 90% homogeneous pool of PEGylated IGFBP-4 having one molecule 40kDa mPEG2 attached to one molecule IGFBP-4. Most probably this pool consists of a mixture of positional isomers, which means that the PEG chain may be linked to different amino acid residues in individual protein molecules. The theoretical molecular weight of mono40kDa-PEG-IGFBP-4 is about 66kDa. Apparently mono4OkDa-PEG-IGFBP-4 runs at 120kDa in SDS-PAGE.
- UnPEGylated IGFBP-4: a homogeneous pool of IGFBP-4 that did not react with the PEG reagent and is recovered e.g. for recycling.

### Example 6

### a) Production of random PEGylated IGFBP-4 (20 kDa)

Wildtype human recombinant IGFBP-4 was randomly PEGylated by the addition of an aqueous solution of N-hydroxysuccinimidyl ester of methoxypoly(ethylene glycol) propionic acid, MW 20,000 (Shearwater Polymers, Inc.; Huntsville, Alabama; thereafter named 20kDa mPEG-SPA). 20kDa mPEG-SPA was added in a molar ratio of 3 molecules PEG per molecule IGFBP-4. The reaction was allowed to proceed at room temperature for 30 minutes and was finally quenched by adding 1M arginine solution (buffered to pH 8.0 with HCl) to a final concentration of 100mM.

The outcome of the PEGylation reaction was optimized for maximal production of monoPEGylated IGFBP-4 with simultaneous minimal consumption of mPEG-SPA reagent by carefully titrating protein and PEG concentrations. For IGFBP-4, yields of the monoPEGylated isoforms is best at elevated protein concentrations (c=5mg/ml or higher) and a 1.5 molar excess of PEGylation reagent.

### b) Purification of 20kDa-PEG-IGFBP-4 isomers

Preparative separation of PEGylation products for biochemical and biological analysis was achieved by size exclusion chromatography on a Sephacryl S 300 column (Pharmacia) in a running buffer consisting of 20mM sodium phosphate pH 7.5 supplemented with 500mM sodium chloride. The 20kDa PEGylated species elute earlier in size exclusion chromatography (SEC) as compared to the unmodified form. This is due to an increased hydrodynamic radius of the molecule.

Eluting fractions were analyzed by SDS-PAGE. In SDS-PAGE proteins are separated according to their molecular weight. PEGylated forms of IGFBP-4 migrated more slowly than the wildtype protein. The speed of migration is inversely correlated with the amount of PEG attached to the protein. Separation was performed on NOVEX 4-12% NuPage gels in a MOPS SDS buffer system.

### Example 7

### Removal of 40kDa mPEG2 or 20kDa mPEG-SPA by ion exchange chromatography

Residual PEGylation reagents that did not react with IGFBP-4 were removed by ion exchange chromatography (IEC) using SP sepharose (Pharmacia). Samples were dialyzed before loading onto the column against 20mM sodium phosphate pH 5.5 to reduce the concentration of sodium, chloride and to adjust to the acidic pH. Under these conditions, free PEG did not bind to the column resin and was detected in the column flow through by a cholorimetric assay as described by Nag, A., et al., Anal. Biochem. 237 (1996) 224-231. Elution of bound protein was performed in a single step with 300mM sodium chloride in 20mM sodium phosphate pH 5.5. Samples were dialyzed against 20mM sodium phosphate pH 7.5, 150mM sodium chloride before storage or further analysis.

### Example 8

### Determination of binding activities by size exclusion chromatography

Distinct quantities of IGFBP-4 (25µg or equivalent amounts of PEGylated isoforms of IGFBP-4) were titrated against known amounts (3,6 and 9µg, respectively) of IGF-1. Residual free IGF-I was quantified by peak integration after separating it from IGF-I/IGFBP-4 complexes by size exclusion chromatography on an HRP75 column (Pharmacia, running buffer consisting of 20mM sodium phosphate pH 7.5 supplemented with 500mM sodium chloride; flow rate 1ml/min).

In this assay, unPEGylated, mono20kDa-PEG-IGFBP-4, poly20kDa-PEG-IGFBP-4 and mono40kDa-PEG-IGFBP-4 showed identical binding of IGF-1. E.g. 96 nmol of mono20kDa-PEG-IGFBP-4 bound 70 nmol of IGF-I.

### Example 9

### Determination of binding parameters with FCS

The ability of IGFBP-4 to form complexes with TAMRA (tetramethylrhodamine) labelled IGF-I was measured by Fluorescence Correlation Spectroscopy (FCS). The assay principle is, that in the absence of a binding partner free fluorescently labelled IGF-I diffuses with a distinct velocity. Addition of IGFBP-4 or PEGylated isoforms leads to complex formation with the labelled IGF-I and a concomitant change in its diffusion velocity. Due to the different diffusion behavior FCS can differentiate bound from freeIGF-I and quantify it. Determination of the amount of bound IGF-I for several concentrations of IGFBP-4 allows one to set up a binding curve and to determine kDa values by curve fitting.

All measurements were performed on a Confocor I ( Zeiss, Jena) at a wavelenght of 543 nm in a buffer consisting of: 100mM HEPES (pH 7.6), 120mM NaCl, 5mM KCl, 1.2mM Mg2SO4; 1mM EDTA, 10mM D(+) Glucose, 15mM sodium acetate, 1% dialyzed bovine serum albumin.

IGF-I binding appeared to be indistinguishable for several IGFBP-4 batches; mono20kDa-PEG-IGFBP-4, poly20kDa-PEG-IGFBP-4, mono40kDa-PEG-IGFBP-4 and wildtype IGFBP-4 showed comparable behavior in terms of complex formation and binding constants (0.34+/- 0.08nM).

### Example 10

### Determination of binding parameters

Inhibitory constants (IC₅₀ values) for wildtype IGFBP-4 and several PEGylated isoforms were determined in Biacore experiments (http://www.biacore.com). Briefly, wildtype IGFBP-4 was immobilized to a Biacore CM5 chip surface by NHS-EDC coupling chemistry as known from the art (http://www.biacore.com). All IGF-I binding experiments were conducted in a commercially available buffer (Biacore HBP-EP; 0.01M Hepes pH 7.4; 0.15M NaCl; 3mM EDTA; 0.005% polysorbat 20 (v/v)). To determine IC₅₀ values, 10nM IGF-I was mixed with eight concentrations from 0.5 to 1000nM of wildtype IGFBP-4 (or of mono20kDa-PEG-IGFBP-4 or mono40kDa-PEG-IGFBP-4) and applied on the chip with immobilized IGFBP-4. Inhibition was measured as a decrease of response units compared to samples of 10nM pure IGF-I in the absence of IGFBP-4. Mono20kDa-PEG-IGFBP-4 and mono40kDa-PEG-IGFBP-4 inhibited IGF-I binding as efficient as wild type control IGFBP-4 with IC₅₀ values of about 4+/- 2nM.

### Example 11

### Inhibition of IGF-I induced IGF-1-receptor phosphorylation by PEGylated IGFBP-4 isoforms

Confluent monolayers of NIH3T3 cells stably expressing human IGF-IR in 3.5 cm dishes were starved in DMEM containing 0.5% dialyzed fetal calf serum. After 48 h, cells were incubated without any hormone or with 5 x 10⁻⁹ M IGF-I; each sample was preincubated with increasing concentrations of IGF-binding proteins or PEGylated isoforms thereof at room temperature for 1 h. After a 10 min stimulation at 37°C, the medium was removed and cells were lysed with 250 µl of lysing buffer (20 mM Hepes, pH 7.5, 150 mM NaCl, 10% glycerol, 1% Nonidet P40, 1.5 mM MgCl₂, 1 mM EGTA (1,2-bis(2-aminoethoxyetna)-N,N,N',N'tetraacidic acid, Aldrich, USA), 10 mM sodium orthovanadate, and protease inhibitor cocktail Complete (Roche Diagnostics GmbH, DE) for 10 min on ice. Subsequently, cells were scraped off the plate and the insoluble material was separated by centrifugation for 20 min at 4°C. The protein concentration of the supernatant was determined using Bicinchoninic acid (Pierce, Rockford, USA; Shihabi, Z.K., and Dyer, R.D., Ann. Clin. Lab. Sci. 18 (1988) 235-239). Equal protein concentration was incubated with the SDS sample buffer (63 mM Tris-HCl, pH 6.8, 3% SDS, 10% glycerol, 0.05% bromphenolblue, 100 mM DTT), boiled for 5 min and loaded on a 7.5% SDS polyacrylamide gel. After electrophoresis the proteins were transferred on a nitrocellulose membrane which first was blocked for 1 h with the 3 % BSA containing PBST (phosphate buffered saline-Tween), then overnight incubated with 1 µg/ml monoclonal anti-phosphotyrosine antibody (Roche Diagnostics GmbH, DE) in PBST that contained 3% BSA. Unbound antibody was removed by extensive washing. The blot was then incubated with 1:10000 diluted anti-mouse IgG-specific antibody conjugated with horse raddish peroxidase (Roche Diagnostics GmbH, DE) and developed.

IGFBP-4, mono20kDa-PEG-IGFBP-4, poly20kDa-PEG-IGFBP-4 and mono40kDa-PEG-IFGBP-4 each displayed equally good inhibitory potential. A three fold molar excess (lowest dose measured) of either isoform blocked receptor phosphorylation induced by 2nM IGF-I completely.

### Example 12

### Inhibition of the growth of tumor cell lines by IGFBP-4 derivatives

The human tumor cell lines PC-3, MDA-MB 231, DU-145, HT29, AsPC-1 and PancTu-1 (from ATCC, American type culture Collection, Rockville, Maryland, U.S.A.) were used to investigate the inhibitory effects of IGFBP-4 derivatives on tumor cell growth. 4000 AsPC-1 cells or 1000 cells of the other cell types were seeded per well in 100 ul RPMI medium containing 10 % FBS (fetal bovine serum) and 1 % glutamine. The cells were cultered in the absence or in the presence of unmodified IGFBP-4 or mono20kDa-PEG-IGFBP-4 for 5 days and cell proliferation was quantified by detecting the cleavage of tetrazolium salts added to the growth medium. Tetrazolium salts are cleaved by mitochondrial dehydrogenase in viable cells (WST-1 assay, PanVera, USA). The growth of the cell lines PC-3, MDA-MB 231, DU-145 was not significantly inhibited by IGFBP-4 derivatives but the growth of the cell lines HT29, AsPC-1 and PancTu-1 was inhibited up to 55 % by IGFBP-4. PEGylated IGFBP-4 is even more potent than unmodified IGFBP-4.

**Table 1:**

| Inhibition of the growth of tumor cell lines by IGFBP-4 derivatives | | | | | | |
|---|---|---|---|---|---|---|
| | PC-3 | MDA-MB 231 | DU-145 | HT29 | AsPC-1 | Panc-Tu1 |
| IGFBP-4 [% Inhibition] | 10 | 0 | 0 | 30 | 40 | 50 |
| Mono20kDa-PEG-IGFBP-4 [% inhibition] | 10 | 0 | 10 | 40 | 50 | 55 |

### Example 13

### Serum kinetics of IGFBP-4 derivatives

SCID mice were injected subcutaneously with a single dose of 1mg/200µl mono20kDa-PEG-IGFBP-4 or mono40kDa-PEG-IGFBP-4 or unPEGylated IGFBP-4 in PBS. Serum samples were collected in a time range from 0.5 to 120h after injection and analyzed for mono20kDa-PEG-IGFBP-4 or mono40kDa-PEG-IGFBP-4 or unPEGylated IGFBP-4 by Western Blotting with an anti-IGFBP-4 antibody (United Biomedical Inc., USA) after affinity purification or by ELISA. Affinity purification was performed by coupling biotinylated IGF-I to streptavidin coated magnetic beads (Roche Diagnostics GmbH, DE) and precipitating IGFBP-4 derivatives out of the respective serum samples by magnetic separation. Bound protein was eluted by heating in SDS sample buffer and separated by SDS PAGE. Proteins were transferred to a PVDF-membrane and detected by an IGFBP-4 specific antibody. Quantification of bands corresponding to IGFBP-4 derivatives was performed by a LumiImager device (Roche).

ELISA testing was performed by capturing PEGylated proteins with a biotinylated monoclonal antibody against PEG (Cheng T. et al.,Bioconjugate Chem. 10 (1999) 520-528) bound to a streptavidin coated microtiter plate and specifically detecting IGFBP-4 with a polyclonal IGFBP-4 antiserum (labeled with peroxidase) produced from rabbits.

Serum levels of mono40kDa-PEG-IGFBP-4 peaked after 24 hours (>75µg/ml) and remained elevated for up to 120h. In comparison, unPEGylated or monoPEG₂₀-IGFBP-4 showed substantially lower peak levels (12 or 35µg/ml, respectively) and a much faster clearance. UnPEGylated IGFBP-4 levels returned to baseline after already 2 hours. AUC is significantly increased by PEGylation and PEGylation with 40 kDa PEG results in significantly higher serum levels for a longer period of time than observed for the 20 kDa PEG derivative of IGFBP-4.

Daily application of mono20kDa-PEG-IGFBP-4 or mono40kDa-PEG-IGFBP-4 or unPEGylated IGFBP-4 led to accumulation in serum of treated mice of mono40kDa-PEG-IGFBP-4 only. Serum levels of over 300µg/ml were achieved at the end of a three-week study.

### Example 14

### Antitumorigenic effect of IGFBP-4 derivatives in the PancTu-1 orthotopic pancreas cancer model

In vitro expanded PancTu-1 tumor cells were removed (0.05% Trypsin-EDTA) from culture flasks and transferred into 50 ml culture medium (RPMI 1640) at the day of injection, washed once (300 x g, 10 min), resuspended in PBS, additionally washed with PBS and filtrated. Cell concentration and cell size were determined and concentration of cells adjusted with PBS to a cell titer of 6.6 x10⁷/ml.

Tumor cells in a volume of 15 µl (= 1.0 x 10⁶ cells) were injected under visible control into the duodenal lobe of the pancreas through the serosa towards the pancreas tissue of 8-10 weeks old female SCID mice (C.B-17) with a body weight of at least about 20 g. Thereafter the pancreas was then gently relocated to the abdominal cavity and the peritoneum incision closed using continuos suture (4-0 vicryl). The skin was adapted and closed with 3-4 wound clips.

Starting on day seven after inoculation with tumor cells two groups of animals with 8 animals per group were treated with either mono20kDa-PEG-IGFBP-4 or mono40kDa-PEG-IGFBP-4. The i.p administered daily dose of 1 mg protein in 0.2 ml PBS was normalized to the protein content of the sample by determination of the absorption of the protein moiety at 280 nm. A third group of 8 animals was treated only with PBS. After 21 days of treatment blood samples were taken from each animal and the primary tumor volume and the tumor weight of each animal was determined. The pancreatic tumor marker CA19.9, a carbohydrate antigenic determinant expressed on a high molecular weight mucin (MUC1) was detected by EIA (ADI, Alpha Diagnostics, Texas, U.S.A.) and Cyfra 21.1 were determined on Elecsys1010 (Roche Diagnostics GmbH, Germany).

Both tumor markers were significanly reduced by treatment with monoPEG₄₀-IGFBP-4 but not by treatment with monoPEG₂₀-IGFBP-4.

Chronic administration of monoPEG₂₀-IGFBP-4 did not inhibit tumor growth. Mean tumor volume at termination was 287 mm³ and very similar to the control group receiving only PBS (226 mm³). In contrast, treatment with monoPEG₄₀-IGFBP-4 reduced tumor growth. Mean tumor volume was calculated at 163 mm³.

**Table 2:**

| **Effect of treatment of PancTu-1 tumor bearing mice with IGFBP-4 derivatives on the serum tumor marker CA19.9** | | | | | |
|---|---|---|---|---|---|
| Group | Treatment | Application | CA19.9 levels (median, U/ml) | Change (%) | CI |
| 2 | Control | i.p. | 127.3 | | |
| 4 | mono20kDa-PEG-IGFBP-4 | i.p. | 108.5 | (-16%) | 0.57-1.24 |
| 6 | mono40kDa-PEG-IGFBP-4 | i.p. | 40.2 | (-66 %) | 0.17-0.79 |

**Table 3:**

| **Effect of treatment of PancTu-1 tumor bearing mice with IGFBP-4 derivatives on the serum tumor marker Cyfra 21.1** | | | | | |
|---|---|---|---|---|---|
| Group | Treatment | Application | Cyfra 21.1 levels (median, ng/ml) | Change (%) | CI |
| 2 | Control | i.p. | 19.3 | | |
| 4 | mono20kDa-PEG-IGFBP-4 | i.p. | 22.6 | (+ 11 %) | 0.63-1.74 |
| 6 | mono40kDa-PEG-IGFBP-4 | i.p. | 10.2 | (- 65 %) | 0.17-0.82 |

### Example 15

### Influence of PEGylated IGFBP-4 on normal kidney cells/kidney organs

Primary tumors and kidney organs were resected and fixed in formalin. Tumors were median divided in two parts and both embedded in one block of paraplast. Both kidney organs were processed (longitudinal and vertical cutting) and embedded. Routine histological staining with hematoxylin and eosin was performed on paraffin.

Chronic treatment with mono20kDa-PEG-IGFBP-4 applied s.c. or i.p. induced moderate to severe histopathological alteration of kidney tissue. Cells belonging to proximal tubules were vacuolated without sign of inflammation and necrosis. These findings were not observed after s.c. or i.p. application of mono40kDa-PEG-IGFBP-4.

### List of References

Byun, D., et al., J. Endocrinology 169 (2001) 135-143
Chelius, D., et al., J. Endocrinology 168 (2001) 283-296
Clark, R., et al., J. Biol. Chem. 271 (1996) 21969-21977
Conover, C.A., et al., J. Biol. Chem. 270 (1995) 4395-4400
Culouscou, J. M., and Shoyab, M., Cancer Res. 51 (1991) 2813-2819
Cunningham, B.C., et al., Science 254 (1991) 821-825
Damon, S. E., et al., Endocrinology 139 (1998) 3456-3464
DE 39 24 705
Diehl, D., et al., J. Cancer Res. Clinical Oncol. 127, Suppl.1 (2001) S54
Duncan, R.J., Anal. Biochem. 132 (1983) 68-73
EP-A 0 473 084
Goodson, R. J., and Katre, N. V., BioTechnology 8 (1990) 343-346
Grasetti, D.R,. and Murray, J.F., J. Appl. Biochem. Biotechnol. 119 (1967) 41-49
Hermanson, G.T., in Bioconjugate Techniques, Academic Press, San Diego (1996) p. 147-148
Hershfield, M.S., et al., New England Journal of Medicine 316 (1987) 589-596
Honda, Y., et al., J. Clin. Endocrinol. Metab. 81 (1996) 1389-1396
http://www.biacore.com
http://www.expasy.ch
Katre, N.V., J. Immunol. 144 (1990) 209-213
Kiefer, M. C., et al., J. Biol. Chem. 267 (1992) 12692-12699
Kinstler, O., et al., Adv. Drug Deliv. Rev. 54 (2002) 477-485
March, J., Advanced Organic Chemistry (1977) 375-376
Meyers, F.J., et al., Clin. Pharmacol. Ther. 49 (1991) 307-313
Miyakoshi, N., et al., Endocrinology 142 (2001) 2641-2648
Mohan, S., et al., Proc. Natl. Acad. Sci. USA 86 (1989) 8338-8342
Monfardini, C., et al., Bioconjug. Chem. 6 (1995) 62-69
Morpurgo, M., et al., J. Bioconjug. Chem. 7 (1996) 363-368
Nag, A., et al., Anal. Biochem. 237 (1996) 224-231
Qin, X., et al., J. Biol. Chem. 273 (1998) 23509-23516
Remington's , Pharmaceutical Sciences, 18th edition, 1990, Mack Publishing Company, edited by Oslo et al. (e.g. pp. 1435-1712)
Schiltz, P. M., et al., J. Bone Mineral Res. 8 (1993) 391-396
Shihabi, Z.K., and Dyer, R.D., Ann. Clin. Lab. Sci. 18 (1988) 235-239
Tanaka, H., et al., Cancer Res. 51 (1991) 3710-3714
US Patent No. 5,382,657
US Patent No. 5,672,662
US Patent No. 5,932,462
van den Berg, C.L., et al., Europ. J. Cancer 33 (1997) 1108-1113
Veronese, F.M., Biomaterials 22 (2001) 405-417
WO 94/01451
WO 94/22466
Wojchowski, D.M., et al., Biochim. Biophys. Acta 910 (1987) 224-232
Yu, H., and Rohan, T., J. Natl. Cancer Inst. 92 (2000) 1472-1489

## Claims

1. A conjugate consisting of insulin-like growth factor binding protein 4 (IGFBP-4) and one or two poly(ethylene glycol) group(s), said poly(ethylene glycol) group(s) having an overall molecular weight of from 30 to 40 kDa .

2. A conjugate according to claim 1, **characterized in that** the poly(ethylene glycol) group(s) is/are branched poly(ethylene glycol) group(s).

3. A conjugate according to claim 1 or 2, **characterized in that** the conjugate is linked via primary amino group(s) of IGFBP-4.

4. A conjugate according to claim 1, **characterized in that** the conjugate is linked via cysteine 110 and/or cysteine 117 of IGFBP-4.

5. A conjugate according to claims 1 to 3 containing one poly(ethylene glycol) group.

6. A method for the preparation of a conjugate comprising an insulin-like growth factor binding protein-4 (IGFBP-4) and one or two poly(ethylene glycol) group(s), said poly(ethylene glycol) group(s) having an overall molecular weight of from 30 to 40 kDa, said method comprising reacting the IGFBP-4 with activated (poly (ethylene) glycol) under conditions such that said (poly(ethylene) glycol) is chemically bound to said IGFBP-4 via primary amino groups or thiol groups of IGFBP-4.

7. A pharmaceutical composition comprising a conjugate of claims 1 to 4 and a pharmaceutically acceptable carrier.

8. A pharmaceutical composition comprising a conjugate according to claims 1 to 4 for the manufacture of a medicament for the treatment of cancer.

## Patentansprüche

1. Konjugat, das aus Insulin-ähnlichem Wachstumsfaktor-bindenden Protein 4 (IGFBP-4) und ein oder zwei Poly(ethylenglycol)-Gruppen besteht, wobei die Poly(ethylenglycol)-Gruppe(n) ein Gesamtmolekulargewicht von 30 bis 40 kDa aufweist/aufweisen.

2. Konjugat nach Anspruch 1, **dadurch gekennzeichnet, dass** die Poly-(ethylenglycol)-Gruppe(n) eine verzweigte Poly(ethylenglycol)-Gruppe ist/ verzweigte Poly(ethylenglycol)-Gruppen sind.

3. Konjugat nach Anspruch 1 oder 2, **dadurch gekennzeichnet, dass** das Konjugat über eine primäre Aminogruppe/primäre Aminogruppen von IGFBP-4 verknüpft ist.

4. Konjugat nach Anspruch 1, **dadurch gekennzeichnet, dass** das Konjugat über Cystein 110 und/oder Cystein 117 von IGFBP-4 verknüpft ist.

5. Konjugat nach einem der Ansprüche 1 bis 3, welches eine Poly-(ethylenglycol)-Gruppe enthält.

6. Verfahren zur Herstellung eines Konjugats, das ein Insulin-ähnliches Wachstumsfaktor-bindendes Protein 4 (IGFBP-4) und ein oder zwei Poly-(ethylenglycol)-Gruppen umfasst, wobei die Poly(ethylengtycol)-Gruppe(n) ein Gesamtmolekulargewicht von 30 bis 40 kDa aufweist/aufweisen, wobei das Verfahren die Umsetzung des IGFBP-4 mit aktiviertem Poly-(ethylenglycol) unter solchen Bedingungen umfasst, dass das Poly-(ethylenglycol) über primäre Aminogruppen oder Thiolgruppen von IGFBP-4 chemisch an das IGFBP-4 gebunden wird.

7. Pharmazeutische Zusammensetzung, umfassend ein Konjugat nach Anspruch 1 bis 4 und einen pharmazeutisch annehmbaren Träger.

8. Pharmazeutische Zusammensetzung, umfassend ein Konjugat nach Anspruch 1 bis 4, zur Herstellung eines Medikaments für die Behandlung von Krebs.

## Revendications

1. Conjugué composé de la protéine 4 de liaison du facteur de croissance insulinomimétique (IGFBP-4) et d'un ou de deux groupes poly(éthylène glycol), ledit ou lesdits groupe(s) poly(éthylène glycol) possédant un poids moléculaire global de 30 à 40 kDa.

2. Conjugué selon la revendication 1, **caractérisé en ce que** le ou les groupe(s) poly(éthylène glycol) est/sont un ou des groupe(s) poly(éthylène glycol) ramifié(s).

3. Conjugué selon la revendication 1 ou 2, **caractérisé en ce que** le conjugué est lié *via* un ou des groupe(s) aminé(s) primaire(s) de l'IGFBP-4.

4. Conjugué selon la revendication 1, **caractérisé en ce que** le conjugué est lié *via* la cystéine 110 et/ou la cystéine 117 de l'IGFBP-4.

5. Conjugué selon les revendications 1 à 3, contenant un groupe poly(éthylène glycol).

6. Procédé de préparation d'un conjugué comprenant une protéine 4 de liaison du facteur de croissance insulinomimétique (IGFBP-4) et un ou deux groupe(s) poly(éthylène glycol), ledit ou lesdits groupe(s) poly(éthylène glycol) possédant un poids moléculaire global de 30 à 40 kDa, ledit procédé comprenant la réaction de l'IGFBP-4 avec un poly(éthylène glycol) activé dans des conditions telles que le poly(éthylène glycol) est chimiquement lié à ladite IGFBP-4 *via* des groupes aminés primaires ou des groupes thiols de l'IGFBP-4.

7. Composition pharmaceutique comprenant un conjugué selon les revendications 1 à 4 et un support pharmaceutiquement acceptable.

8. Composition pharmaceutique comprenant un conjugué selon les revendications 1 à 4 pour la fabrication d'un médicament destiné au traitement du cancer.
